# EUROPEAN PATENT APPLICATION

(11) **EP 4 257 981 A1**
(43) Date of publication of application: **11.10.2023**
(21) Application number: 22167229.8
(22) Date of filing: 07.04.2022
(51) Int. Cl.: G01N 33/543

(54) **SENSING DEVICE WITH IMPROVED DETECTION SENSITIVITY FOR DETECTING THE PRESENCE OF A PREDEFINED CHEMICAL, BIOLOGICAL OR BIOCHEMICAL ENTITY IN A FLUID SAMPLE**

(71) Applicant: CSEM Centre Suisse d'Electronique et de Microtechnique SA - Recherche et Développement, 2002 Neuchâtel (CH)
(72) Inventor: ZINGGELER, Marc, 4052 Basel (CH); SCHÄR, Sandra, 3360 Herzogenbuchsee (CH)
(74) Representative: e-Patent SA

(57) **Abstract**

The invention concerns a sensing device (1, 30) comprising a sensing surface (16) intended to detect the presence of a predefined detectable entity formed in an assay compartment (12) and arranged so as to be switchable between a first configuration, in which it is in fluidic communication with the assay compartment (12) during the formation of the predefined detectable entity, and a second configuration, in which it is in fluidic communication with a detection compartment (4) arranged so that no fluid can freely flow between the assay compartment (12) and the detection compartment (4).

## Description

### Technical Field

The invention concerns a sensing device comprising a substrate having a sensing surface intended to detect the presence of a predefined detectable entity which is electrically, optically or magnetically detectable and which is representative of the presence of at least one predefined chemical, biological or biochemical entity in a fluid sample.

More specifically, the invention concerns such a sensing device further comprising an assay compartment and a detection compartment arranged in such a way that no fluid can freely flow from one to another.

The invention also concerns a method for carrying out the detection of a predefined entity in a fluid sample, based on the use of a sensing device having the above-mentioned features.

### State of the art

Sensing devices as described above are already known in the state of the art such as, in particular, lateral flow assays (LFA, or LFIA for lateral flow immunoassays) or vertical flow assays (VFA).

There is a shift from traditional diagnostic tests performed in the centralized clinical laboratory setting to the decentralized point of care (POC) setting. The term POC implies the close vicinity to the patient and promises to provide physicians with (almost) instantaneous diagnostic information in order to make faster and better informed decisions. While POC devices could allow patients in developed countries to personally monitor their own health quantitatively at home, they could enable life-saving and affordable diagnostic procedures for infectious disease in the low resource clinical setting in the developing countries.

Immunochromatographic sensing devices, such as LFA or VFA are the most widely used devices for POC testing. These devices consist of an assembly of different porous materials and enable the users to perform analytical assays by following a very simple procedure. In most cases the addition of a sample to the sample well is sufficient. The sample is then transported passively by capillary action along/through the different materials that contain the required reagents to complete the analytical binding assay. In a very common format the sample flows first through a conjugate pad that contains a labelled detection antibody that is released into the sample and binds to the analyte. The formed couple is then transported further through a test strip and bound to the immobilized capture antibody in the test region. In most cases the formed couples in the test region are read-out by optical detection (e.g. by eye) of the label contained on the detection antibody.

To enhance the sensitivity of such devices signal amplification strategies are of high interest. To achieve this, the label on the detection antibody is not measured directly but used to interact with an added amplification reagent to generate an excess of measurable species. A widely used strategy is to use enzyme labelled detection antibodies (e.g. horseradish peroxidase - HRP) followed by the addition of an enzyme substrate (e.g. 3,3',5,5'-Tetramethylbenzidine - TMB) to produce a measurable product (e.g. oxidized TMB).

A recent review of such systems is provided in: Calabria, D.; Calabretta et al. Recent Advancements in Enzyme-Based Lateral Flow Immunoassays. Sensors 2021, 21, 3358.

In all known prior art devices signal amplification and read-out are performed on the test strip which strongly limits the achievable Signal/Noise ratio (SNR) due to the following key problems:
1/ Addition of the (liquid) signal amplification reagent to immunochromatographic tests is not easily implemented because of the (fully) soaked compartments, and
2/ The test compartment (e.g. nitrocellulose (NC) flow strip) which was in contact with the sample and the other assay components possess high background and an uncontrolled environment for signal amplification and read-out.

Publication WO 2004/010143 A2 describes a conventional sensing device of the above-mentioned type, having limitations in the sensitivity of the analyte detection, while publications WO 2019/208961 A1 and WO 2019/245744 A1 disclose different approaches to improve the sensitivity of such conventional sensing devices.

However, all these proposed solutions lead to a poor SNR as far as the conditions of implementation of the corresponding detection step are not optimized and can still be improved.

On the basis of what precedes, it appears that a need still exists for a sensing device which would permit to improve assay sensitivity by still increasing SNR.

### Disclosure of the invention

An aim of the invention is to propose a structure for a sensing device, allowing the latter to fulfil the conventional requirements in terms of ease of use, selectivity and reliability, which would further improve its sensitivity.

More specifically, the invention relates to a sensing device as mentioned above, characterized by the fact that it further comprises a mechanical switching mechanism including a guiding surface with respect to which the substrate can be guided so as to move its sensing surface between the assay compartment and the detection compartment, such that the sensing surface can be in fluidic communication either with the assay compartment or with the detection compartment.

More preferably, it is provided that the detection compartment is arranged such that an amplification reagent can be brought in contact with the sensing surface when the latter is in fluidic communication with the detection compartment.

More particularly, it can be provided, in that case, that the detection compartment comprises a fluid inlet arranged for allowing the introduction of a detection fluid containing or able to release the amplification reagent, the detection compartment being designed such that the detection fluid is able to flow from the fluid inlet to the sensing surface when the latter is in fluidic communication with the detection compartment.

Based on the above features, the sensitivity of the sensing device according to the invention is not only improved thanks to the use of an amplification reagent, but also by increasing SNR due to the fact that the conditions of the detection step are optimized, by switching the sensing surface into a specific detection configuration wherein it is not in contact anymore with the assay compartment and with the remaining free entities which are not representative of the presence of the analyte target in the sample fluid.

According to a first conventional approach, it is possible to provide
that the sensing device comprises a reaction compartment, at least a part of which defines the assay compartment, having a sample inlet for the introduction of the fluid sample and containing a free conjugated entity which can be brought into contact with the fluid sample and which is adapted to react exclusively with the at least one predefined chemical, biological or biochemical entity so as to create a combined entity, the reaction compartment being designed so as to allow the fluid sample to flow from the sample inlet to the assay compartment,
that the sensing surface carries an immobilized capture entity adapted to capture the combined entity so as to create an immobilized precursor on the sensing surface when the latter is in fluidic communication with the assay compartment, and
that the amplification reagent is adapted to react with the immobilized precursor, when the sensing surface is in fluidic communication with the detection compartment.

In that case, the reaction between the amplification reagent and the immobilized precursor directly creates the predefined detectable entity.

According to a first alternative embodiment, following a first competitive approach, it is possible to provide
that the sensing device comprises a reaction compartment, at least a part of which defines the assay compartment, the reaction compartment:
   - having a sample inlet for the introduction of the fluid sample,
   - being designed so as to allow the fluid sample to flow from the sample inlet to the assay compartment, and
   - containing a free labelled entity which can be brought into contact with the fluid sample and which is adapted to react exclusively with an immobilized capture entity carried by the sensing surface, when the latter is in fluidic communication with the assay compartment, so as to create an immobilized labelled precursor on the sensing surface, the immobilized capture entity being also adapted to react with the at least one predefined chemical, biological or biochemical entity so as to create a non-labelled combined entity on the sensing surface, and
that the amplification reagent is adapted to react with the immobilized labelled precursor when the sensing surface is in fluidic communication with the detection compartment.

In this case, the free labelled entity, provided in a known predefined quantity, competes with the at least one predefined chemical, biological or biochemical entity to react with the immobilized capture entity. Thus, the more the sample fluid contains the at least one predefined chemical, biological or biochemical entity, the weaker is the detection signal as less particles of the free labelled entity could react with the immobilized capture entity on the sensing surface.

According to a second alternative embodiment, following a second competitive approach, it is possible to provide
that the sensing device comprises a reaction compartment, at least a part of which defines the assay compartment, the reaction compartment:
   - having a sample inlet for the introduction of the fluid sample,
   - being designed so as to allow the fluid sample to flow from the sample inlet to the assay compartment, and
   - containing a free labelled entity which can be brought into contact with the fluid sample and which is adapted to react both with the at least one predefined chemical, biological or biochemical entity and with an immobilized capture entity carried by the sensing surface, when the latter is in fluidic communication with the assay compartment, so as to create an immobilized labelled precursor on the sensing surface, and
that the amplification reagent is adapted to react with the immobilized labelled precursor, when the sensing surface is in fluidic communication with the detection compartment.

In this case, the at least one predefined chemical, biological or biochemical entity competes with the immobilized capture entity to react with the free labelled entity which is provided in a known predefined quantity. Thus, the more the sample fluid contains the at least one predefined chemical, biological or biochemical entity, the weaker is the detection signal as less particles of the free labelled entity are available to react with the immobilized capture entity on the sensing surface.

On a general basis, it might be advantageous to provide that the reaction compartment comprises at least one porous carrier for allowing the fluid sample to flow from the sample inlet to the assay compartment.

Further, it might be advantageous to provide that the sensing surface defines a portion of an electrode.

In this case, it might be preferable that the electrode comprises at least two electrical contacts enabling the detection of the predefined detectable entity to be carried out by implementation of an electrochemical process on the sensing surface.

Generally, it might be advantageous to provide that the sensing surface is made of a solid material having a hydrophilic polymer coating and/or that the sensing surface is a part of a porous carrier.

According to a preferred embodiment, the sensing device might be a LFA sensing device or a VFA sensing device.

In the conventional approach and in the first alternative embodiment, it might be preferable to provide that the immobilized capture entity is an antibody. In this case, it might be advantageous to provide that the free conjugated entity is an antibody containing an enzyme label. Further, when the detection compartment contains the amplification reagent such that the latter can be released by the detection fluid, the amplification reagent might preferably be an enzyme substrate, and the predefined detectable entity an enzyme product.

Generally, the substrate might advantageously be arranged to be movable at least according to a translation movement or to a rotation movement.

Furthermore, the substrate might preferably comprise, or be assembled with a support comprising, a control surface which is accessible for the user such that the latter can act on it to move the electrode.

An additional aim of the present invention is to provide a method for detecting the presence of at least one predefined chemical, biological or biochemical entity in a fluid sample by implementation of a sensing device according to the preceding features, so as to detect the presence of a predefined detectable entity, which is electrically, optically or magnetically detectable and which is representative of the presence of the at least one predefined chemical, biological or biochemical entity, the method comprising the steps consisting in:
a) preparing the sensing device such that its sensing surface is in fluidic communication with the assay compartment,
b) introducing a sample fluid in the assay compartment,
c) waiting for a first predefined incubation time,
d) moving the sensing surface from the assay compartment to the detection compartment, such that it is in fluidic communication with the detection compartment,
e) introducing the amplification reagent in the detection compartment,
f) waiting for a second predefined incubation time, and
g) carrying out a measure to detect the presence of the predefined detectable entity.

According to a preferred embodiment of the above method, moving step d) might preferably include a translation movement or a rotation movement.

At least one optional washing step of the sensing surface might be provided between steps c) and d) and/or between steps d) and e).

### Brief description of the drawings

Further details of the invention will appear more clearly upon reading the description below, in connection with the following figures which illustrate:
- Fig. 1: schematic diagram illustrating the general principle of the present invention; illustration of the general construction;
- Figs. 2a and 2b: similar schematic illustrations of a sensing device according to a first preferred embodiment of the present invention, in two respective configurations corresponding to two different steps of a measurement operation;
- Figs. 3a and 3b: similar schematic illustrations of a sensing device according to a second preferred embodiment of the present invention, in two respective configurations corresponding to two different steps of a measurement operation;
- Figs. 4a and 4b: schematic diagrams illustrating the results of measurement operations, respectively carried out with a sensing device according to the prior art and with a sensing device according to the present invention, and
- Figs. 5 to 8: simplified illustrations of four different embodiments of a sensing device according to the present invention.

### Embodiments of the invention

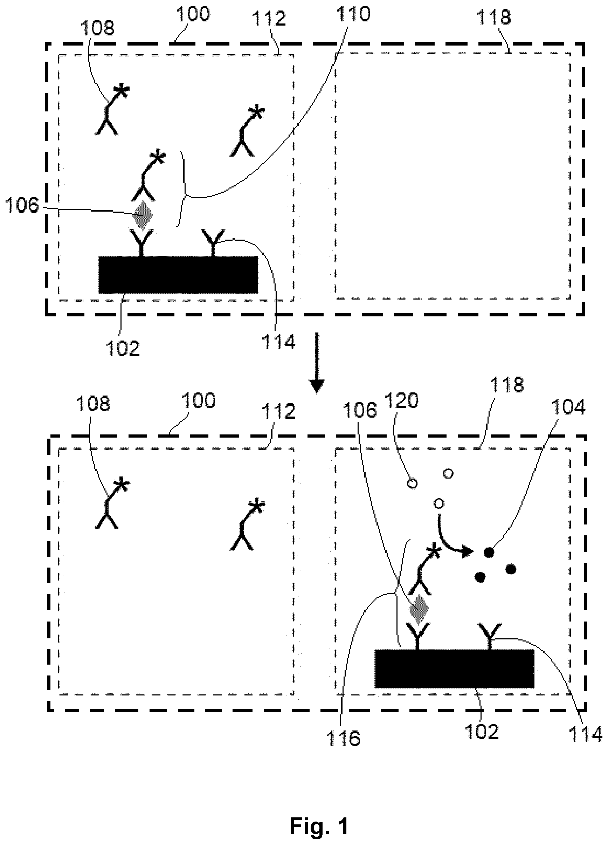

Figure 1 is a schematic diagram illustrating the general principle of the present invention.

The invention generally relates to a sensing device 100 comprising a sensing surface 102 intended to detect the presence of a predefined detectable entity 104 which is electrically, optically, or magnetically detectable and which is representative of the presence of at least one predefined chemical, biological or biochemical entity 106 in a fluid sample.

The sensing device 100 can be used to carry out measurements related to different fields of application, for instance for conducting medical diagnosis, in the course of a drug development, to carry out a quality control, a drug abuse control, a doping control or an environment control.

The sensing surface 102 is advantageously made of a solid material like a polymer, a glass, or a metal for instance.

The sensing device 100 further comprises a reaction compartment, having a sample inlet for the introduction of the fluid sample and containing a free conjugated entity 108 which can be brought into contact with the fluid sample, and which is adapted to react exclusively with the predefined chemical, biological or biochemical entity 106 so as to create a combined entity 110, according to a conventional approach. Alternative embodiments based on a competitive approach will be briefly described later.

Typically, the free conjugated entity 108 might be immobilized on a dry surface of the reaction compartment during the fabrication of the sensing device 100, so as to be released when the dry surface is contacted by the fluid sample. It is also possible to provide that the free conjugated entity 108 is added in the reaction compartment by introduction of a carrier fluid through the sample inlet or through any additional inlet without going beyond the scope of the present invention as defined in the appended claims.

The predefined chemical, biological or biochemical entity 106 can be any molecule or species (e.g. protein, virus, cell) for which a specific capture entity exists, while the free conjugated entity 108 can be any molecule or other species (e.g. antibody, aptamer, DNA, RNA) that is able to specifically bind the predefined chemical, biological or biochemical entity 106, and preferably labelled and/or containing an entity enabling signal amplification (e.g. enzyme). Thus, the sample fluid can be a body fluid from a human being or from an animal, like the whole blood, blood plasma, blood serum, urine, saliva, sweat, tears. Alternatively, the sample fluid can be a sample from a cell culture or other in-vitro model (for instance an organ-on-chip system), a food or beverage, or water (drinking, waste or environment).

The reaction compartment further includes an assay compartment 112 and is designed so as to allow the fluid sample to flow from the sample inlet to the assay compartment 112. The assay compartment 112 is a space that can hold fluid (e.g. porous material, fluidic channel, well).

It should be noted that the one skilled in the art will encounter no particular difficulty to implement a shape and a size for the reaction compartment which will fulfil his needs without going beyond the scope of the present invention. At least a part of the reaction compartment might define the assay compartment. Thus, in its most basic form and size, the reaction compartment might be simply limited to an assay compartment.

The sensing surface 102 carries an immobilized capture entity 114 adapted to capture the combined entity 110 so as to create an immobilized precursor 116 on the sensing surface 102.

The immobilized capture entity 114 can be any molecule or other species (e.g. antibody, aptamer, DNA, RNA) that is also able to specifically bind the predefined chemical, biological or biochemical entity 106.

Once the sample fluid is introduced in the reaction compartment, it flows to the assay compartment 112 before an incubation period takes place, during which the predefined chemical, biological or biochemical entity 106 is bound by the immobilized capture entity 114 which is present at the sensing surface 102 and by the free conjugated entity 108 which is added/released into the sample fluid. After the incubation period, the concentration of formed immobilized precursor 116 on the sensing surface 102 is a function of the concentration in the sample fluid of the predefined chemical, biological or biochemical entity 106.

An optional washing step might be implemented here, by using a standard washing or buffer solution.

According to the invention, the sensing device 100 further comprises a detection compartment 118 arranged such that no fluid can freely flow between the assay compartment 112 and the detection compartment 118. The detection compartment 118 can be any space that can hold fluid (e.g. porous material, fluidic channel, well).

The sensing surface 102 is advantageously switchable between a first configuration (upper half of Fig. 1), in which it is in fluidic communication with the assay compartment 112, so as to allow the creation of the immobilized precursor 116, and a second configuration (lower half of Fig. 1), in which it is in fluidic communication with the detection compartment 118. When the sensing surface 102 is switched from its first configuration to its second configuration, it is separated from the assay compartment 112. Furthermore, during this operation, the major part of the free conjugated entity 108 which is not bound is left behind, in the assay compartment 112 and is not transferred into the detection compartment 118.

If no fluid can freely flow from the assay compartment to the detection compartment, it is still possible that a negligible quantity of fluid is transferred from the assay compartment to the detection compartment as the sensing surface is moved from one compartment to the other without any measurable impact on the implementation of the present invention.

Another optional washing step might be implemented here, by using a standard washing or buffer solution.

It should be noticed that the sensing surface 102 might be switched from a configuration to the other either manually, in response to a predefined action of a user on the sensing device 100, or automatically, if for instance a timing device would be used to trigger a corresponding switching mechanism after a predefined period. The one skilled in the art will encounter no particular difficulty to implement the corresponding technical details as a function of his specific needs and without going beyond the scope of the present invention.

The detection compartment 118 may comprise a fluid inlet, arranged for allowing the introduction of a detection fluid containing or able to release an amplification reagent 120, and is designed such that the detection fluid is able to flow from the fluid inlet to (and all over) the sensing surface 102, when the latter is in its second configuration, the amplification reagent 120 being adapted to react with the immobilized precursor 116, during a new incubation period, to create the predefined detectable entity 104 on the sensing surface 102 or in its close vicinity. Typically, the amplification reagent 120 may react with a portion of the free conjugated entity 108 which is included in the immobilized precursor 116. Consequently, in most cases but in a non-limiting manner, the amplification reagent 120 can be any molecule or species (e.g. enzyme substrate) known that can form a measurable species by interaction with the free conjugated entity 108.

Alternatively, the detection compartment 118 might be pre-filled with an amplification reagent 120 prior to the measurement operation, preferably as a liquid and during the fabrication process of the sensing device 100, so as to be released and react with the immobilized precursor 116 when the sensing surface 102 is brought in fluidic communication with the detection compartment 118.

The formed species, which is the predefined detectable entity 104, is then measured in the detection compartment 118 in real time (i.e. kinetic measurement) or after a specified incubation time (end-point measurement) using known methods (e.g. optically or electrically). The measured signal is a function of the concentration in the sample fluid of the predefined chemical, biological or biochemical entity 106 and, as such, enables biomarker quantification.

Any adapted known measurement methods might be implemented, preferably electrochemical (for instance potentiometry, amperometry, voltammetry, impedance spectroscopy) or, alternatively, optical (for instance absorption, emission like chemiluminescence).

As previously stated, according to a first alternative embodiment, following a first competitive approach, it is possible to provide that the reaction compartment contains a free labelled entity which can be brought into contact with the fluid sample and which is adapted to react exclusively with an immobilized capture entity carried by the sensing surface, when the latter is in fluidic communication with the assay compartment, so as to create an immobilized labelled precursor on the sensing surface, the immobilized capture entity being also adapted to react with the at least one predefined chemical, biological or biochemical entity so as to create a non-labelled combined entity on the sensing surface. The amplification reagent is then adapted to react with the immobilized labelled precursor when the sensing surface is in fluidic communication with the detection compartment.

In this case, the free labelled entity, provided in a predefined quantity, competes with the at least one predefined chemical, biological or biochemical entity to react with the immobilized capture entity. Thus, the more the sample fluid contains the at least one predefined chemical, biological or biochemical entity, the weaker is the detection signal as less particles of the free labelled entity could react with the immobilized capture entity on the sensing surface.

According to a second alternative embodiment, following a second competitive approach, it is possible to provide that the reaction compartment contains a free labelled entity which can be brought into contact with the fluid sample and which is adapted to react both with the at least one predefined chemical, biological or biochemical entity and with an immobilized capture entity carried by the sensing surface, when the latter is in fluidic communication with the assay compartment, so as to create an immobilized labelled precursor on the sensing surface. The amplification reagent is then adapted to react with the immobilized labelled precursor, when the sensing surface is in fluidic communication with the detection compartment.

In this case, the at least one predefined chemical, biological or biochemical entity competes with the immobilized capture entity to react with the free labelled entity which is provided in a predefined quantity. Thus, the more the sample fluid contains the at least one predefined chemical, biological or biochemical entity, the weaker is the detection signal as less particles of the free labelled entity are available to react with the immobilized capture entity on the sensing surface.

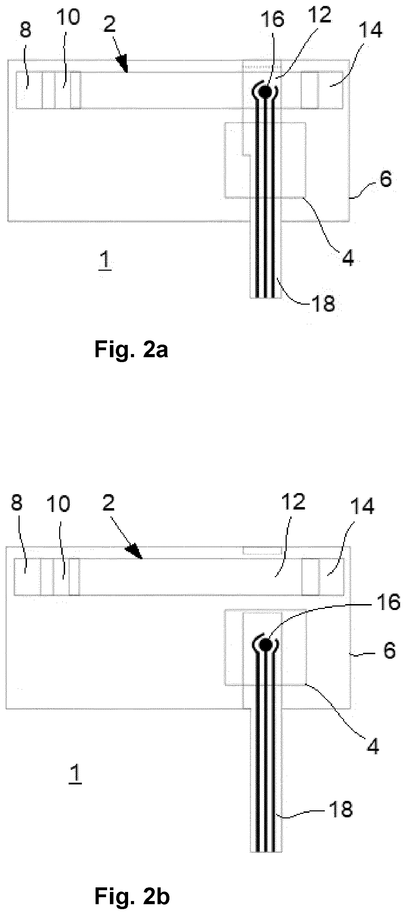

An implementation example of main embodiment of the present invention is schematically illustrated in Figs. 2a and 2b, which are similar schematic illustrations of a sensing device 1 according to a first preferred embodiment of the present invention, in two different configurations respectively corresponding to the first configuration of its sensing surface and to its second configuration.

More precisely, sensing device 1 takes here the form of a lateral flow immunoassay (LFIA) device with electrochemical read-out. However, unlike conventional LFIA, the sensing device 1 according to the invention comprises a reaction compartment 2, preferably including a porous carrier, and a separate detection compartment 4, both built in a common cartridge 6 here, in a non-limiting manner.

Lateral flow immunoassays adapted to detect the presence of more than one predefined entity are also known, and the present invention is not limited to the detection of one entity only. The one skilled in the art will be able to adapt the present teaching to implement a test device for the simultaneous detection of more than one predefined entity without any particular difficulty and without going beyond the scope of the present invention. This includes the application of POCT (Point Of Care Tests) for biomarkers detection in body fluids (blood, serum, saliva, urine, tear and so on) by direct immunoassay, sandwiches immunoassay or competitive immunoassay, detection of pollutants (pesticides, herbicides) in the environmental monitoring, detection of toxins, antibiotic residues, pesticides residues for food quality control.

The cartridge 6 can preferably be made of injection molded plastic for instance. But it can also alternatively be made of 3D-printed or machined plastic, or machined metal without going beyond the scope of the invention. It can be made in one piece or by the assembly of several distinct parts, for instance a bottom part assembled with a cover part. In that case, the two parts can be clipped together.

Like typical LFIA, the reaction compartment 2 comprises a sample pad 8, defining a sample dropping area from which a dropped sample fluid is intended to start flowing in the reaction compartment 2. The sample pad 8 can preferably be made of cellulose fibers. Alternatively, it can be made of glass fibers or woven meshes. It can optionally contain blocking agents to be released into the sample fluid.

The sensing device 1 further comprises, following the flow direction of the sample, a conjugate pad 10, defining a conjugate area intended to include at least one free conjugated entity, which is adapted to react exclusively with the predefined chemical, biological or biochemical entity to be detected so as to create a predefined combined entity. The conjugate pad 10 can preferably be made of glass fibers. Alternatively, it can be made of cellulose fibers or woven meshes. It can optionally further contain blocking agents to be released into the sample fluid.

In a well-known manner, more than one free conjugated entity can be provided when more than one entity has to be detected in the sample.

The predefined chemical, biological or biochemical entity to be detected can be, for instance, chosen in the group comprising preferably proteins, or DNA or RNA, metabolites, drugs, toxins, cells, bacteria and viruses, while the matching free conjugated entity can be chosen in the group comprising preferably proteins too (for instance antibodies), or aptamers, molecularly imprinted polymers, DNA or RNA, cells, bacteria and viruses, entities which are conjugated directly or indirectly to a label, preferably enzymes (more preferably HRP), catalysts or particles containing measurable species (for instance, coupled molecules - like an antibody coupled to an enzyme, microor nanoparticles - like gold nanoparticles with surface immobilized antibodies and enzymes, or enzyme loaded hydrogel particles that contains surface immobilized antibodies).

The sensing device 1 may further comprise a regulating pad (not illustrated) to control the flow (speed) through the reaction compartment 2. Such a flow regulating pad might preferably be made of nanocellulose.

Downstream the conjugate pad 10, or the optional regulating pad if there is one, the reaction compartment 2 comprises an assay compartment 12 which may preferably comprise a nitrocellulose membrane or a fluidic channel or well. Alternatively, the assay compartment 12 may comprise a cellulose fibers membrane.

An absorption pad 14 is typically provided then, preferably made of cellulose fibers, to pull the sample fluid through the assay compartment 12.

Fig. 2a and 2b further illustrate a preferred embodiment of the sensing device 1 in which the latter comprises a sensing surface 16 defining a portion of a substrate, taking the form of an electrode 18 here.

In Fig. 2a, the sensing surface 16 is in a first configuration in which it is in fluidic communication with the assay compartment 12.

The sensing surface 16 can be made of solid plastic, metal, glass or ceramic. The whole electrode 18 can preferably be a screen-printed electrode (for instance made of carbon on a PET foil). More preferably, the sensing surface 16 may comprise a hydrophilic polymer coating to reduce non-specific adsorption of assay or sample components and carries at least one immobilized capture entity as mentioned above. As already stated, the immobilized capture entity can preferably be a protein or, in alternative, DNA or RNA, a metabolite, a drug, a toxin, a cell, a bacteria or a virus.

As previously explained, once the sample fluid is introduced in the reaction compartment 2, it flows to the assay compartment 12 and a predefined incubation period takes place, during which the predefined chemical, biological or biochemical entity is bound by the immobilized capture entity which is present at the sensing surface 16 and by the free conjugated entity which is released from the conjugate pad 10 into the sample fluid.

After the incubation period, an immobilized precursor is formed on the sensing surface 16, the concentration of which is a function of the concentration in the sample fluid of the predefined entity to be detected.

After the end of the incubation period, the sensing surface 16 is switched to its second configuration as illustrated in Fig. 2b, by being simply pulled here, in which it is in fluidic communication with the detection compartment 4.

A detection fluid containing an amplification reagent can then be introduced in the detection compartment 4, so as to flow from the corresponding fluid inlet (not illustrated) to the sensing surface 16, the amplification reagent being adapted to react with the immobilized precursor, during a new incubation period, to create the predefined detectable entity on the sensing surface 16 or in its close vicinity. Alternatively, an amplification reagent can be contained in the detection compartment 4, preferably in a dry state, so as to be released by the detection fluid which could be an adapted conventional buffer fluid, for instance.

The amplification reagent can preferably be an enzyme substrate (for instance TMB), or a redox active species, a dye or a dye precursor, a chemical entity adapted to release a measurable species from a label provided on the free conjugated entity.

The formed species, which is a predefined detectable entity (depending on the nature of the entity to be detected in the sample, and on the choice of the free conjugated entity, of the immobilized capture entity, and of the amplification reagent), is then measured in the detection compartment 4 in real time or after a specified incubation time with any of the previously mentioned measuring methods adapted to the nature of the detectable entity (and thus to its physical and/or chemical properties).

The predefined detectable entity can preferably be a redox active species, or a dye or, more generally, an electron or a photon.

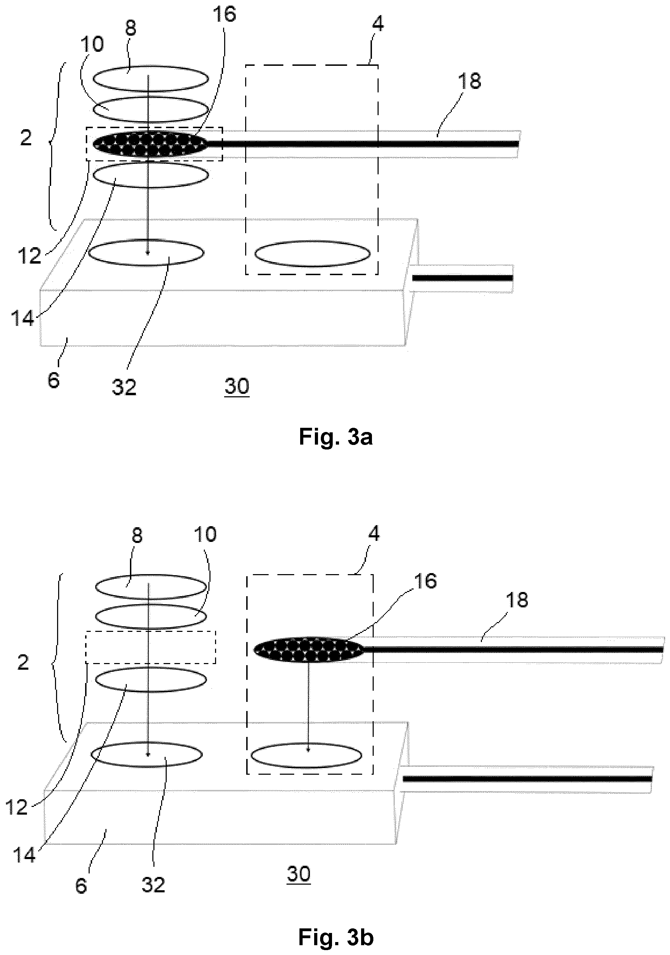

An additional implementation example of the present invention is schematically illustrated in Figs. 3a and 3b, which are similar schematic illustrations of a sensing device 30 according to a second preferred embodiment of the present invention, in two different configurations respectively corresponding to the first configuration of its sensing surface and to its second configuration. The same reference numerals are used as in Fig. 2a and 2b for designing same components of the sensing devices 1 and 30, from a functional point of view, for easing the comprehension of this additional exemplary embodiment.

More precisely, sensing device 30 takes here the form of a vertical flow assay (VFA) device with electrochemical read-out. According to the present invention, the sensing device 30 according to the present embodiment also comprises a reaction compartment 2 and a separate detection compartment 4, both built in a common cartridge 6, in a non-limiting manner.

The different components of the reaction compartment 2 are arranged vertically instead of being arranged horizontally like in the embodiments of Fig. 2a and 2b.

As previously explained, once a sample fluid is introduced in the reaction compartment 2, by preferably being dropped on its sample pad 8, it flows to the assay compartment 12 in which the sensing surface 16 is positioned in its first configuration, as illustrated in Fig. 3a, through the conjugate pad 10, and a predefined incubation period takes place, during which the predefined chemical, biological or biochemical entity is bound by the immobilized capture entity which is present at the sensing surface 16 and by the free conjugated entity which is released from the conjugate pad 10 into the sample fluid.

The sample fluid is pulled through the sensing surface 16 by the absorption pad 14 and by gravity. In this case, the sensing surface 16 is advantageously fluid permeable, the corresponding electrode 18 taking for instance the form of a perforated screen-printed electrode.

After the incubation period, an immobilized precursor is formed on the sensing surface 16, the concentration of which is a function of the concentration in the sample fluid of the predefined entity to be detected.

An optional waste pad 32 can be provided below the absorption pad 14 to absorb a possible excess of sample fluid flowing out of the absorption pad 14.

After the end of the incubation period, the sensing surface 16 is switched to its second configuration as illustrated in Fig. 3b, by being simply pulled, in which it is in fluidic communication with the detection compartment 4.

A detection fluid containing or able to release an amplification reagent can be introduced in the detection compartment 4 so as to contact the sensing surface 16, the amplification reagent being adapted to react with the immobilized precursor, during a new incubation period, to create the predefined detectable entity on the sensing surface 16 or in its close vicinity. For instance, the detection fluid can be directly dropped on the sensing surface 16 in the case of a VFA.

The formed species is then measured in the detection compartment 4 in real time or after a specified incubation time with any of the previously mentioned measuring methods adapted to the nature of the detectable entity.

More generally, the switching mechanism for switching the sensing surface from its first configuration to its second configuration can be a pushing or a pulling mechanism, or a turning mechanism, or a fold or unfolding mechanism. Furthermore, the actuation of the switching mechanism can be carried out manually or automatically without going beyond the scope of the present invention. The movable mechanism can include a support for the sensing surface, for instance, and the support might comprise a control surface that may be actuated manually, by an operator, or automatically, to switch the sensing surface.

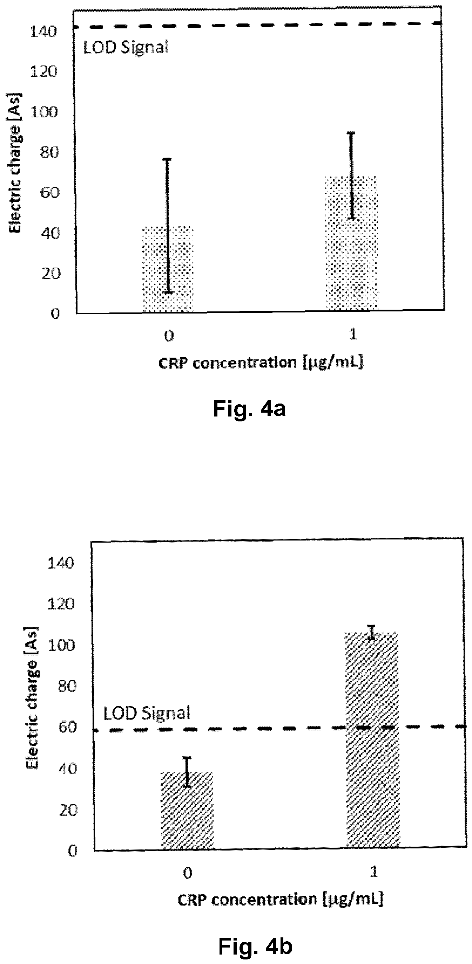

Figs. 4a and 4b are schematic diagrams illustrating the results of electrochemical read-out operations, for detecting CRP (C-Reactive Protein, an important inflammatory biomarker) in a sample fluid, respectively carried out with a sensing device according to the prior art and with a sensing device according to the present invention as described in connection with Fig. 2a and 2b.

A capture antibody (cAb) against CRP was immobilized on the sensing surface of a screen-printed carbon electrode and a detection antibody (dAb) was prepared on the conjugate pad of the LFA device. The sensing surface of the electrode was brought in contact with the assay compartment during the assay procedure (i.e. application of CRP sample and incubation for 10 min). The dAb was labelled with HRP enzyme which allows signal amplification and electrochemical assay read-out by oxidizing the substrate TMB. To reproduce the conditions of the state-of-the-art device, TMB used as an amplification reagent is added directly to the reaction compartment (Fig. 4A). In the case of the sensing device according to the present invention, the electrode is moved to the detection compartment, to switch its sensing surface from its first configuration to its second configuration, before allowing an incubation with TMB (Fig. 4B). After an incubation time of 10 min, the oxidized TMB is electrochemically reduced at the electrode sensing surface and the current recorded.

A comparison of the measurement results, i.e. between the diagrams of Fig. 4a and of Fig. 4b, clearly illustrates two main problems associated with the classical electrochemical LFA, namely high background and low signal, the measurement being notably improved with the device of the invention (Fig. 4b). In the shown example the SNR of the test was increased by factor 13.

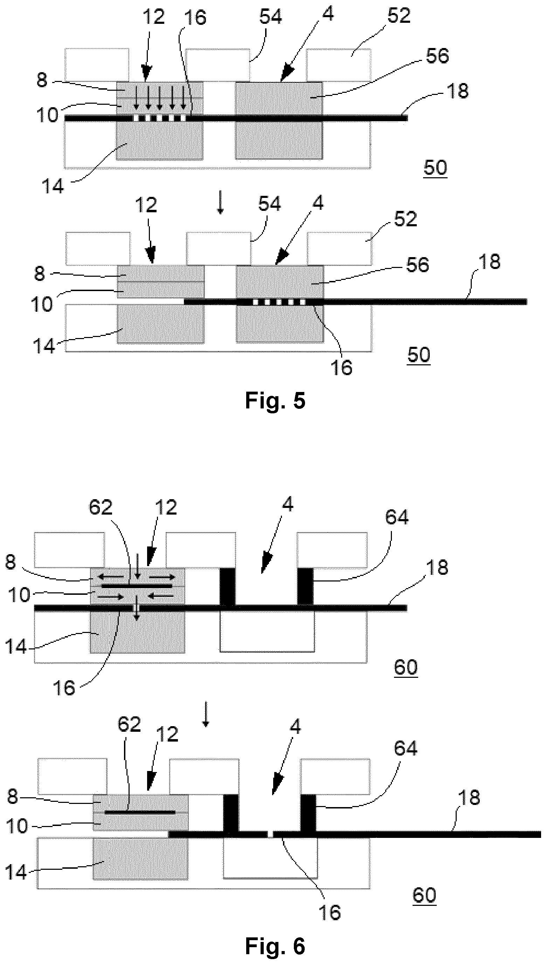

Figure 5 is a simplified illustration of an additional implementation example of a sensing device 50 according to the present invention, in two different configurations respectively corresponding to the first configuration of its sensing surface and to its second configuration. The same reference numerals are used as in Fig. 3a and 3b for designing same components of the sensing devices 30 and 50, from a functional point of view, for easing the comprehension of this additional exemplary embodiment.

The sensing device 50 is very similar to the sensing device 30 which was previously described.

More precisely, the sensing device 50 is of the VFA type and has its assay compartment 12 and its detection compartment 4 composed of porous materials that absorbed the added fluids and hold them in place, in particular to prevent spilling-over to other areas of the device). Note that the reaction compartment 2 is not illustrated here as far as it is nearly limited to the assay compartment 12.

The electrode 18 comprises a sensing surface 16 which contains multiple perforations enabling vertical flow through it (see arrows) of added fluids, at least the sample fluid and optionally at least one washing solution.

The switching between the first and second configurations of the sensing surface 16 is performed by pulling the electrode 18 which might advantageously be fixed in its first position (with the sensing surface 16 in fluidic communication with the assay compartment 12) by a "soft stop", for instance clamped in between the different pads of the device 50, by the housing 52.

It should be noted that the sensing device 50 comprises at least one guiding surface to guide the electrode 18 when it is moved from a position to the other. This guiding surface can be implemented as portions of pads and/or of the housing 52.

The electrode 18 is thus pulled to move the sensing surface 16 in its second configuration, i.e. in fluidic communication with the detection compartment 4, which position might be characterized by a "hard stop", for instance mechanically engaging structures that prevent the user to pull the electrode 18 any further without damaging the sensing device 50.

After switching, an optional washing/buffer solution might be added before the amplification reagent is dropped through the additional inlet 54.

Enough volume of the amplification reagent might be added to make sure that the reagent pad 56 is fully soaked or nearly.

Figure 6 is a simplified illustration of an additional implementation example of a sensing device 60 according to the present invention, in two different configurations respectively corresponding to the first configuration of its sensing surface and to its second configuration. The same reference numerals are used as in Fig. 3a and 3b for designing same components of the sensing devices 30 and 60, from a functional point of view, for easing the comprehension of this additional exemplary embodiment.

The sensing device 60 is very similar to the sensing device 30 which was previously described.

More precisely, the sensing device 50 is of the VFA type and has an electrode 18 the sensing surface 16 of which has only a single centered perforation.

A liquid tight layer 62 is provided in-between the sample pad 8 and the conjugate pad 10. The liquid tight layer 62 induces lateral flow in the sample pad 8 and in the conjugate pad 10 to increase flow time and achieve sufficient washing in regions that would not properly be washed by vertical flow with only a single central perforation.

This embodiment also illustrates a different structure of the detection compartment 4 which is formed here by an empty space enclosed by a sealing 64 on the liquid holding side.

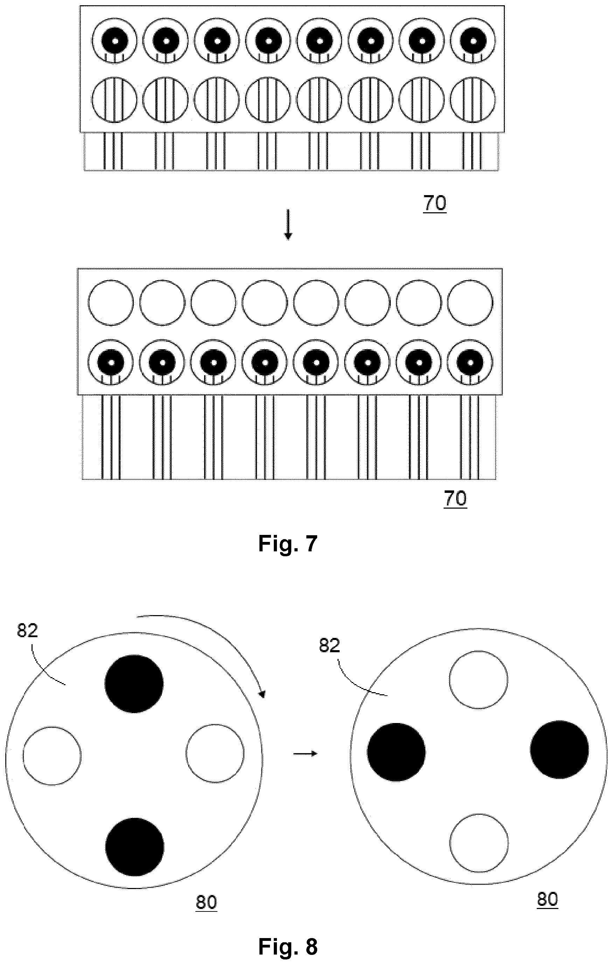

Figure 7 is a simplified face illustration of an alternative embodiment of a sensing device 70 according to the present invention enabling multiplexing, i.e. measuring different samples or markers in parallel on the same device. Some of the wells might also be used to control measurements in a similar way as that of the control lines in a LFA.

Figure 8 is a simplified face illustration of an alternative embodiment of a sensing device 80 according to the present invention, in two different configurations respectively corresponding to the first configuration of its sensing surface and to its second configuration.

According to the present embodiment, a different structure is implemented for the switching mechanism. More precisely, the sensing surface is moved by way of a rotation movement about an axis which is substantially perpendicular to the general main plane of the sensing device 80, to move between the assay compartment and the detection compartment. For instance, the electrode might be tightened to the lid 82 of the housing and a rotation of the lid 82, here by 90 degrees in a illustrative and non-limiting way, would lead to a rotation of the sensing surface from the assay compartment to the detection compartment.

As an alternative to mechanical stop mechanisms the user might also be guided to the right positions by optical means (e.g. appearing colors or signs in a window of the housing as soon as the right partition are reached).

The rotation could also be performed automatically. In this case a rotation mechanism might start by itself after a certain time or might be induced by a simple "push bottom" action performed by the user.

It appears from the preceding description that the sensing device according to the present invention offers better sensitivity with respect to prior sensing devices, while being still easy to manufacture on a large scale and being a practical solution for point-of-care assessments.

Indeed, thanks to the features of the sensing device according to the invention, it is possible to overcome the above-mentioned limitations of the prior art with a new device concept including a sensor that can be switched between two states. In the first state, the sensor surface, which contains immobilized capture antibodies (cAb) or other capture molecules, is in fluidic communication with the assay compartment. In this state a classical binding assay is performed (e.g. by adding the sample to the sample pad of the device). After the binding assay has been completed, the system is switched to the second state by bringing the sensing surface into the detection compartment which is located at another position of the sensing device. Ideally this can be achieved by the user in a very simple and controlled way (e.g. simple pulling mechanism). With this action most of the interfering background components are left behind in the assay compartment (e.g. on the flow pad, or reaction compartment) and the signal amplification and read-out can be performed in a controlled and "fresh" (uncontaminated) environment. The detection compartment may be pre-filled with the signal amplification reagent or is filled after the sensing surface has been moved in. Ideally, the sensing surface contains a special anti-fouling coating to minimize the amount of background components which are transferred to the detection compartment. The sensing surface may also be washed (e.g. by adding a washing solution while it is still in the assay compartment, or by pulling it through a washing compartment) before moving to the detection compartment. The key concept is the switching of states to decouple the assay and the signal amplification/read-out steps during implementation of a measure.

Finally, key advantages or the present invention are that:
- classical binding assays can be performed using existing components (flow pads and reagents); high potential for mass manufacturing of the sensing devices and low entry barrier for clinical adoption due to known assay procedures, and
- higher SNR can be achieved by the de-coupling of the binding assay and the signal amplification/read-out through the switching of device states; enhanced sensor performance enabling device application for the measurement of various biomarkers (low-abundance targets, measurements in complex body fluids) at the POC.

The one skilled in the art will encounter no particular difficulty to adapt the present teaching to his needs by implementing different entities systems in connection with a sensing device according to the invention, comprising a sensing surface that can be switched between two configurations, without going beyond the scope of the invention as defined by the appended claims.

## Claims

1. Sensing device (1, 30) comprising:
- an assay compartment (12) and a detection compartment (4) arranged in such a way that no fluid can freely flow from one to another, and
- a substrate including a sensing surface (16) intended to detect the presence of a predefined detectable entity which is electrically, optically or magnetically detectable and which is representative of the presence of at least one predefined chemical, biological or biochemical entity in a fluid sample,
**characterized in that** it further comprises a mechanical switching mechanism including a guiding surface with respect to which said substrate can be guided so as to move said sensing surface (16) between said assay compartment (12) and said detection compartment (4), such that said sensing surface (16) can be in fluidic communication either with said assay compartment (12) or with said detection compartment (4).

2. Device (1, 30) according to claim 1, **characterized in that** said detection compartment (4) is arranged such that an amplification reagent can be brought in contact with said sensing surface (16) when the latter is in fluidic communication with said detection compartment (4).

3. Device (1) according to claim 2, **characterized in that** said detection compartment (4) comprises a fluid inlet arranged for allowing the introduction of a detection fluid containing or able to release said amplification reagent, said detection compartment (4) being designed such that said detection fluid is able to flow from said fluid inlet to said sensing surface (16) when the latter is in fluidic communication with said detection compartment (4).

4. Device (1, 30) according to claim 2 or 3, **characterized**
**in that** it comprises a reaction compartment (2), at least a part of which defines said assay compartment (12), having a sample inlet for the introduction of said fluid sample and containing a free conjugated entity which can be brought into contact with said fluid sample and which is adapted to react exclusively with said at least one predefined chemical, biological or biochemical entity so as to create a combined entity, said reaction compartment (2) being designed so as to allow said fluid sample to flow from said sample inlet to said assay compartment (12),
**in that** said sensing surface (16) carries an immobilized capture entity adapted to capture said combined entity so as to create an immobilized precursor on said sensing surface (16) when the latter is in fluidic communication with said assay compartment (12), and
**in that** said amplification reagent is adapted to react with said immobilized precursor, when said sensing surface (16) is in fluidic communication with said detection compartment (4).

5. Device (1, 30) according to claim 2 or 3, **characterized**
**in that** it comprises a reaction compartment (2), at least a part of which defines said assay compartment (12), said reaction compartment:
- having a sample inlet for the introduction of said fluid sample,
- being designed so as to allow said fluid sample to flow from said sample inlet to said assay compartment (12), and
- containing a free labelled entity which can be brought into contact with said fluid sample and which is adapted to react exclusively with an immobilized capture entity carried by said sensing surface (16), when the latter is in fluidic communication with said assay compartment (12), so as to create an immobilized labelled precursor on said sensing surface (16), said immobilized capture entity being also adapted to react with said at least one predefined chemical, biological or biochemical entity so as to create a non-labelled combined entity on said sensing surface (16), and
**in that** said amplification reagent is adapted to react with said immobilized labelled precursor when said sensing surface (16) is in fluidic communication with said detection compartment (4).

6. Device (1, 30) according to claim 2 or 3, **characterized**
**in that** it comprises a reaction compartment (2), at least a part of which defines said assay compartment (12), said reaction compartment:
- having a sample inlet for the introduction of said fluid sample,
- being designed so as to allow said fluid sample to flow from said sample inlet to said assay compartment (12), and
- containing a free labelled entity which can be brought into contact with said fluid sample and which is adapted to react both with said at least one predefined chemical, biological or biochemical entity and with an immobilized capture entity carried by said sensing surface (16), when the latter is in fluidic communication with said assay compartment (12), so as to create an immobilized labelled precursor on said sensing surface (16), and
**in that** said amplification reagent is adapted to react with said immobilized labelled precursor, when said sensing surface (16) is in fluidic communication with said detection compartment (4).

7. Device (1, 30) according to any of claims 4 to 6, **characterised in that** said reaction compartment (2) comprises at least one porous carrier for allowing said fluid sample to flow from said sample inlet to said assay compartment (12).

8. Device (1, 30) according to any of the preceding claims, **characterized in that** said sensing surface (16) defines a portion of an electrode (18) which preferably comprises at least two electrical contacts enabling said detection of said predefined detectable entity to be carried out by implementation of an electrochemical process on said sensing surface (16).

9. Device (1, 30) according to any of the preceding claims, **characterized in that** said sensing surface (16) is made of a solid material having a hydrophilic polymer coating.

10. Device (1, 30) according to any of the preceding claims, **characterized in that** said sensing surface (16) is a part of a porous carrier.

11. Device (1, 30) according to any of the preceding claims, **characterized in that** it is a LFA sensing device or a VFA sensing device.

12. Device (1, 30) according to any of claims 4, 5 or 7 to 11, **characterized in that** said immobilized capture entity is an antibody.

13. Device (1, 30) according to claims 4 and 12, **characterized in that** said free conjugated entity is an antibody containing an enzyme label.

14. Device (1, 30) according to claim 13, **characterized in that** said detection compartment (4) contains said amplification reagent such that the latter can be released by said detection fluid, **in that** said amplification reagent is an enzyme substrate, and **in that** said predefined detectable entity is an enzyme product.

15. Device (1, 30) according to any of the preceding claims, **characterized in that** said substrate is arranged to be movable at least according to a translation movement or to a rotation movement.

16. Device (1, 30) according to any of the preceding claims, **characterized in that** said substrate comprises, or is assembled with a support comprising, a control surface which is accessible for the user such that the latter can act on it to move said substrate.

17. Method for detecting the presence of at least one predefined chemical, biological or biochemical entity in a fluid sample by implementation of a sensing device (1, 30) according to any of claims 1 to 15, so as to detect the presence of a predefined detectable entity, which is electrically, optically or magnetically detectable and which is representative of the presence of said at least one predefined chemical, biological or biochemical entity, the method comprising the steps consisting in:
a) preparing said sensing device (1, 30) such that its sensing surface (16) is in fluidic communication with said assay compartment (12),
b) introducing a sample fluid in said assay compartment (12),
c) waiting for a first predefined incubation time,
d) moving said sensing surface (16) from said assay compartment (12) to said detection compartment (4), such that it is in fluidic communication with said detection compartment (4),
e) introducing said amplification reagent in said detection compartment (4),
f) waiting for a second predefined incubation time, and
g) carrying out a measure to detect the presence of said predefined detectable entity.

18. Method according to claim 17, **characterized in that** the moving step d) includes a translation movement or a rotation movement of said sensing surface (16).

19. Method according to claim 17 or 18, **characterized in that** at least one washing step of said sensing surface (16) is implemented between steps c) and d) and/or between steps d) and e).
